# EUROPEAN PATENT SPECIFICATION

(11) **EP 1 608 660 B1**
(45) Date of publication and mention of the grant of the patent: **10.10.2007**
(21) Application number: 03719080.8
(22) Date of filing: 31.03.2003
(51) Int. Cl.: C07D 491/22

(54) **PROCESS FOR PREPARING TOPOTECAN FROM 10-HYDROXY-4-(S) CAMPTOTHECIN**
VERFAHREN ZUR HERSTELLUNG VON TOPOTECAN AUS 10-HYDROXY-4-(S) CAMTOTHECIN
PROCEDE POUR PREPARER DU TOPOTECAN A PARTIR DE 10-HYDROXY-4-(S)-CAMPTOTHECINE

(43) Date of publication of application: 28.12.2005
(73) Proprietor: COUNCIL OF SCIENTIFIC AND INDUSTRIAL RESEARCH, New Delhi 110 001 (IN)
(72) Inventor: PURI, Satish, Chander, Jammu, Jammu & Kashmir (IN); HANDA, Geeta, Jammu, Jammu & Kashmir (IN); DHAR, Kanaya, Lal, Jammu, Jammu & Kashmir (IN); SURI, Om, Parkash, Jammu, Jammu & Kashmir (IN); QAZI, Ghulam, Nabi, Jammu, Jammu & Kashmir (IN)
(74) Representative: Smyth, Gyles Darren
(86) International application number: PCT/IN2003/000108
(87) International publication number: WO 2004/087715

(56) References cited:
- WO-A-92/05785
- US-A- 5 004 758
- LAUDER W. JONES AND HERBERT F. WHALEN: "The Action of Acid Chlorides upon Trimethylamine" J. AM. CHEM. SOC., vol. 47, 1925, pages 1343-52, XP002268859

## Description

### Field of the invention

The present invention relates to the use of dihalomethanes as reagents for the preparation of Topotecan {4-(S)-10 (dimethylamino)-methyl-4-ethyl 4, 9 dihydroxy1-H-pyrano [3'4': 6,7] indolizino-[1,2-b]quinoline-3,14 (4H,12H)dione} of formula I from 10-hydroxycamptothecin. The invention discloses the rationale use of dichloromethane under solid-liquid phase transfer catalysis, which can behave both as solvent and a reactant when it serves as a source for C-1 unit for amino-alkylation of 10-hydroxy-4- (S) camptothecin.

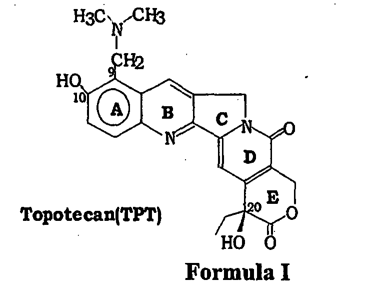

### Background of the invention

Topotecan (TPT) [9-(dimethylamino)-methyl]-10-hydroxy- (4S)-camptothecin) which is of the chemical structure of Formula I, is under clinical trials and its chemotherapeutic efficacy appears to be very promising. (Slichenmyer, W.J., Rowinsky, E.K., Donehower, R.C., J. Natl. Cancer Inst., 85:271 1993) Topotecan is one of the new class agents those targets topoisomerase I (Topo I) and stabilize the DNA-Topo-1 complex, ultimately resulting in the cell death. The rationale for the use of Topotecan in chronic lymphocytic leukemia (CLL) is based on the finding of Top-I being elevated in the lymphocytes of patients with this disease (O'Brien, S., Kantarjian, H., Ellis, A., Zwelling, L., Estey, E., Keating, M., Cancer, 75 (5) 104-1995).

WO 92/05 785 reports a process for the preparation of topotecan from 10-hydroxy-4(S)-camptothecin.

Camptothecin and congeners represent a clinically very useful class of anticancer agents. The discovery that the (S) enantiomer of camptothecin (CPT), a compound isolated from the bark, leaves and fruit of *Camptotheca acuminata*, can kill cell through selective poisoning of the human enzyme topoisomerase-I bound to its substrate DNA, was important breakthrough for its development as an anticancer agent, The intact 20-(S)-lactone form of CPT first isolated over 30 years ago, (Wall, M.E., Wani, M. C., Cooke, C. E., Palmer, K.H., McPhail, A.T., J. Am. Chem. Soc. 88 3888 1966) can bind non-covalently to the complex formed by topoisomerase-I and DNA thus inhibiting the resealing of broken DNA backbone. The intrinsic ability of CPT to trap this complex has been clearly linked to antitumor activity especially for tumors over-expressing topoisomerase-I such as colorectal and cervical cancer (Tahimoto, C.H., Wright, H.J., Arbuck, S.G., Biochim. Biophys. Acta 1400 107 1998 and Iyer, L., Ratain, M.J., Cancer Chemotherapy Pharamcol 42 31 1998). Nanomolar concentrations of the drug are in fact sufficient to cause DNA damage *in vivo,* which becomes irreversible following its collision with DNA processing machineries. This collision event produces irreparable damage to the DNA (Hsiang, Y.H., Lihou, M.G., Liu, L.F., Cancer Res. 49 5077 1989) and finally results in cell death (Tsao, Y.P., Arpa, D., Liu, L.F., Cancer Res. 52 1823 1992 and Holm, C., Covey, J. M., Kerrigan, D., Pommier, Y., Cancer Res. 49 6365 1989). CPT is not an optimal drug as it exhibits very limited water solubility in addition to severe toxicity and erratic absorption. Despite its serious side effect, it has become such a promising antitumor agent that extensive research, considering both pharmokinetics and pharmacodynamic has led to the successful development of new closely related compounds. 10-Hydroxy- (20S) camptothecin (HCPT) was shown to possess therapeutic effect on liver carcinoma, leukemia, cancers associated with head and neck. 10-Hydroxy-(20S)-camptothecin was reported to show improved antitumor activity and was found to be ten times more potent against P-388 and 1210 mouse leukemia than the parent camptothecin and was found also to be less toxic. The hydrophobicity of CPT precluded its development as a clinical agent and necessitated the use of the hydrophilic synthetic congeners in various phases of clinical trails. 10-Hydroxycamptothecin has been isolated as a minor compound (0.002%) from the extract of stem wood of *C.acuminata* by Wall, M.E., & Wani, M.C., (J. Org. Chem. 34.1364 1969) and *Ophirrhiza mungos* Linn (Tafur, S., Nelson, J. D., Delong, D.C. and Svobodo, G.H., Lloydia 39 261 1976). Wani, M.C., (J. Med. Chem. 23(5) 554 1980) reported the total synthesis of (dl)-9-[(dimethylamino)-methyl]-10-hydroxycamptothecin with the ring E intact involving a number of steps. However, the method gives a low yield and therefore is only of academic value.

Kingbury, W. D., (J. Med. Chem. 37 98 1991) converted CPT to HCPT by reduction-oxidation sequence using platinum catalyst to afford mixture of compounds including 10-acetoxycamptothecin and unreacted CPT. This method of preparation is not economically viable. Among HCPT congeners Camptosar (Irinotecan HCl CPT-11 by Pharmacia & Upjohn) and Hycamtin (Topotecan HCl TPT SmithKline Beecham Pharmaceutical) have been approved for the treatment of metastatic colorectal carcinoma and small cell lung cancer along with refractory ovarian cancer.

New potent and water soluble derivatives have been synthesized and are now in clinical studies while other potent drugs are in pre-clinical stage as second generation camptothecin. Functionalization at 7,9,10, positions is compatible with increase in activity as shown by the 9-amino-20- (S)-camptothecin, Lurtotecan (G-I147211) (Takimoto, C.H., Wright, J. S., Arbuck, G., Chemother. Pharmacol. 42 1400 1998) and Exetecan (Dx-8951) (Mitsui, I., Kumazawa, E., Hirota, Y., Aonuma, M., Sugumori, M., Ohsuki, S., Uoto, K., Ejima, A., Tersawa, H., Sato, K., Jpn. J Cancer Res. 88 760 1995).

### Objects of the invention

The main object of the present invention is to present an improved process for the preparation of Topotecan-HCl from 10-Hydroxycamptothecin by aminoalkylation, using dihalomethane, viz. dichloromethane, dibromomethane, or diidomethane as a reagent, by unconventional Mannich reaction.

Another object of the invention is to obtain better yield than by classical methods of inducting C-1 unit using low boiling, low density and less toxic reagent, dichloromethane in place of formaldehyde.

Another object of the invention is to carry out reaction under mild conditions at low pressure and at room temperature with high reactivity and to prevent at the same time polyalkylation, which is a problem for electron-rich phenolic substrate.

Another object is to obtain Eschenmoser's salts (N-methyl-N-methylenmethaniminium salts) which follow the reaction pathway in which soluble and reactive "ion pair" formed after gegenion exchange from potassium *ortho* phenolate and Eschenmoser's salts which eventually collapses to give ortho-attacked products.

Another object of the invention is to compare the behavior of protic/aprotic solvents for ortho-regiospecific monoalkylated products.

### Summary of the invention

Accordingly the present invention provides a process for preparing 9-[(dimethylamino)-methyl]-10-hydroxycamptothecin (topotecan) of the formula 1 below 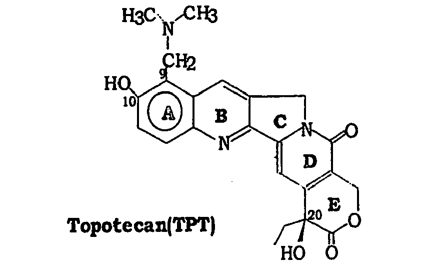 from 10-hydroxy-20-(S)-camptothecin (HCPT) dissolved in an organic solvent, the process comprising *ortho-*regloselective aminomethylation of HCPT with dimethylamine, using a dihalomethane which behaves both as solvent and a reactant, under soud-liquid phase transfer catalysis along with a solid base catalyst in suspension form, and under stirring and at room temperature, filtering the solid product obtained and washing the obtained solid product, evaporating the solvent and purifying the residue to obtain the desired product.

In one embodiment of the invention, the dihalomethane is selected from the group consisting of dichloromethane, dibromomethane and diidomethane.

In another embodiment of the invention, the solvent medium is selected from the group consisting of methylene halides, toluene, acetonitrile, dimethylformamide and any mixture thereof.

In yet another embodiment of the invention, the solid base catalyst is selected from the group consisting of potassium carbonate, sodium carbonate, ammonium carbonate, lithium carbonate and hydrated potassium carbonate.

In a further embodiment of the invention, the stirring is done at a pressure in the range of 69-124 kPa (10-18 psi) for a period of 4-8 hours.

In a further embodiment of the invention, the reaction is carried out at a temperature in the range of 25 °C-45 °C and on a rotary shaker at 220-250 rpm.

In another embodiment of the invention, the product topotecan obtained is in the form of an acetate or a hydrochloride salt by freeze drying

In a further embodiment of the invention, the acetate of topotecan is converted to the pure hydrochloride salt thereof by adding dilute aqueous hydrochloric acid to the solution of acetate salt of topotecan followed by lyophilization.

In another embodiment of the invention, filtered residue is washed with ethyl acetate.

The obtained residue is preferably purified by repeated recrystallization or by distillation.

### Detailed description of the invention

Purification of the product may be effected by conventional chromatography or by repeated crystallization and finally characterized by physico-chemical techniques.

10-Hydroxycamptothecin up to 99% purity was stirred with anhydrous potassium carbonate along with dimethylamine and dihalomethane at room temperature 25 °C for 5 hours. The reaction was monitored by chromatographic techniques TLC, HPLC using different solvent systems at different wavelengths. The formation of the products was also determining by UV scanning. The substrate shows bathochromic shift when treated with dilute base. On TLC substrate (HCPT) shows orange colored spots, where as TLC chromatogram of the products shows yellow spot on UV (254nm) on UV visualization. It is observed that toluene is solvent of choice for electron-rich phenols since it decreases polyalkylation whereas dichloromethane usually gives higher reactivity for substrate bearing electron-withdrawing groups.

The following examples are illustrative and not limiting of the scope of the invention. This description will clearly enable one skilled in the art to make and use the invention and describes several embodiments, adaptations, variations, alternatives and uses of the invention including what we presently believe is the best mode of carrying out the invention.

### Example I

A) 10-Hydroxycamptothecin was prepared by subjecting camptothecin (3.2g 0.0092 mol), 0.8g of Pt⁰ (prepared by pre-reduction of 8g of amorphous PtO₂ in 80ml of HOAc for 1.5hr under 1 atmosphere hydrogen pressure) and acetic acid to 1 atm. of H₂ for 8.5h after which theoretical amount of H₂ absorbed (slightly more than 0.4 1) and uptake of H₂ gets slowed down The reaction mixture was degassed under steam of Helium and filtered through celite and washed with HOAc (20ml). The resulting solution of 1, 2, 6, 7 tetrahydroxy-camptothecin was treated immediately with Pb (OAc)₄ (6.4g 0.014mol) in portions and reaction mixture stirred vigorously under Helium for 30 min. Gummy residue was obtained on evaporation of solvent which was triturated with cold water (100 ml) to produce light brown solid. The solid was collected, washed with cold water and air dried overnight when a mixture of 10-HCPT (44%), 10-AcHOCPT (26%) and unreacted CPT (32%) on HPLC basis was obtained. This crude mixture was combined with 150ml of 50% HOAc and heated under reflux conditions overnight. The reaction mixturewas cooled, concentrated to 20ml and treated with cold water (100ml) to produce precipitate, which is filtered, washed with more cold water and dried to afford 2.1g of solid containing HCPT (70%) AcCPT (1.2%) and CPT (21.3%) on the basis HPLC. Mixture was triturating with 0.5% aq HCl to dissolve the water-soluble. When insoluble CPT was removed by filtration. Water-soluble was extracted with chloroform and crystallized from boiling solution of 20% of MeOH in CHCl₃ by adding EtOAC dropwise until turbidity appeared to obtain pure yellow HCPT which gives orange colored spot on TLC (CHCl₃, acetone, MeOH 7:2:1), C₂₀H₁₆N₂O₅ (m/s 364), mp 268-270°C, UV. λmax. 222, 266, 330 and 382 IR (KBr) 3480 (OH), 1740 (Lactone) and 1655 (pyridone) cm⁻¹; ¹H NMR 0.88 (t, 3, C-18), 1.85 (m, 2, C-19 CH₃), 5.35 (s, 2, C-17), 6.40 (s, C-20 OH), 7.22 (s, 1H, C-14), 7.28 (m, C-11 and C-12), 7.26 (d, 1, C-9), 8.38 (1, s, C-7), 10.3 (s, br, C-10 OH).

### 9-[(Dimethylamino) methyl] 10-hydroxy (20s)-camptothecin (Topotecan)

HCPT (0.364g 0.01mmol) and 40% aqueous dimethylamine (12ml) was added in dichloromethane (50ml) in which anhydrous potassium carbonate (2.17g, 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. Water-soluble were partitioned with petroleum ether (3x50ml) and followed by ethylacetate (3x50ml). The aqueous layer was lyophilized as an off white hydrochloride salt yield 0.236g (65%), C₂₃H₂₃N₃O₅. (m/s.421.44); IR (KBr) 3400, 2960, 1740, 1650, 1590 cm⁻¹; ¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example II

HCPT (0.364g 0.01mmol) and 40% aqueous dimethylamine (12ml) was added in dibromomethane (50ml) in which anhydrous potassium carbonate (2.17g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. Water-soluble were partitioned with petroleum ether (3x50ml) and followed by ethylacetate (3x50ml). The aqueous layer was lyophilized as an off white hydrochloride salt yield 0.244g (67%), C₂₃H₂₃N₃O₅. (m/s.421.44); IR (KBr) 3400, 2960, 1740, 1650, 1590 cm⁻¹; ¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example-III

HCPT (0.364g 0.01 mmol) and 40% aqueous dimethylamine (12ml) was added in diiodomethane (50ml) in which anhydrous potassium carbonate (2.17g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. Water-soluble were partitioned with petroleum ether (3x50ml) and followed by ethylacetate (3x50ml). The aqueous layer was lyophilized as an off white hydrochloride salt yield 0.250g (69%), C₂₃H₂₃N₃O₅. (m/s.421.44); IR (KBr) 3400, 2960, 1740, 1650, 1590 cm⁻¹; ¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s;C-7).

### Example IV

HCPT (0.364g 0.01mol) and 40% aqueous dimethylamine (12ml) was added in dichloromethane (50ml) in which potassium carbonate sesquihydrated (2.48g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. Water-soluble were partitioned with petroleum ether (3x50ml) and then followed by ethylacetate (3x50ml). The aqueous layer was lyophilized as an off white hydrochloride salt; yield 0.218g (60%) C₂₃H₂₃N₃O₅. (m/s.421.44); IR (KBr) 3400, 2960, 1740, 1650, 1590 cm-¹; ¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example-V

HCPT (0.364g 0.01mol) and 40% aqueous dimethylamine (12ml) was added in dichloromethane (50ml) in which anhydrous sodium carbonate (1.44g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. The water-soluble were partitioned with petroleum ether (3x50ml) and then followed by ethylacetate (3x50ml). The aqueous layer was lyophilized as an off white hydrochloride salt; yield 0.124g (34%) C₂₃H₂₃N₃O₅. (m/s.421.44); IR (KBr) 3400, 2960, 1740, 1650, 1590 cm⁻¹;
¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example VI

HCPT (0.364g 0.01mol) and 40% aqueous dimethylamine (12ml) was added in dichloromethane (50ml) in which potassium carbonate (2.78g 20mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. The water-soluble were partitioned with petroleum ether (3x50ml) and then followed by ethylacetate (3x50ml). The aqueous layer was lyophilized as an off white hydrochloride salt; yield 0.196g (54%) C₂₃H₂₃N₃O₅. (m/s.421.44); IR (KBr) 3400, 2960, 1740, 1650, 1590 cm⁻¹;

¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example-VII

HCPT (0.364g 0.01mol) and 40% aqueous dimethylamine (12ml) was added in dichloromethane (50ml) in which lithium carbonate (1.11g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. The water-soluble were partitioned with petroleum ether (3x50ml) and then followed by ethylacetate (3x50ml). The aqueous layer was lyophilized as an off white hydrochloride salt, yield 0.113g (31%), C₂₃H₂₃N₃O₅. (m/s.421.44); IR (KBr) 3400, 2960, 1740, 1650, 1590 cm-¹;
¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2; C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example-VIII

HCPT (0.364g 0.01mol) and 40% aqueous dimethylamine (12ml) in toluene was added in dichloromethane (50ml) in which potassium carbonate (2.17g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. The water-soluble were partitioned with petroleum ether (3x50ml) and then followed by ethylacetate (3x50ml). The aqueous layer was lyophilized as an off white hydrochloride salt, yield 0.149g (41%). C₂₃H₂₃N₃O₅. (m/s.421.44); IR (KBr) 3400, 2960, 1740, 1650, 1590 cm⁻¹;
¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).

### Example-IX

A solution of HCPT (0.364g 0.01mol) and 40% aqueous dimethylamine (12ml) in dimethylformamide was added in dichloromethane (50ml) in which potassium carbonate (2.17g 15mmol) has been suspended. The reaction mixture was stirred at room temperature for 5 hour, then filtered and solid extracted with ethylacetate (20ml). The solvent is evaporated in vacuo giving a residue. The residue was triturated with 0.5% aq HCl (50ml) to dissolve the water-soluble adduct. The water-soluble were partitioned with petroleum ether (3x50ml) and then followed by ethylacetate (3x50ml). The aqueous layer was lyophilized as an off white hydrochloride salt; yield 0.51g (14%). C₂₃H₂₃N₃O₅. (m/s.421.44); IR (KBr) 3400, 2960, 1740, 1650, 1590 cm⁻¹;
¹H NMR (CDCl₃) 1.04 (t, 3, J=7 Hz, C-18), 1.96 (q, 2, J=7 Hz, C-19), 2.01 (s, 3, CH₃CO₂), 2.50 (s, 6, (CH₃)₂NH), 4.20 (s, 2, ArCH₂N), 5.28 (d, 1, J=19 Hz, C-17), 5.29 (s, 2, C-5), 5.50 (d, 1, J=10 Hz, C-17), 7.42 (d, J=9 Hz, C-11), 7.67 (s, 1, C-14), 8.05 (d, J=9 Hz, C-12), 8.51 (s, C-7).
**The main advantages of the present invention are:**
1. Out of three solid bases used for solid-liquid transfer catalysis for ortho-aminomethylation of 10-Hydroxycamptothecin, potassium carbonate appears to be best choice for making 9-[(dimethylamino) methyl]-10-hydroxy- (20s)-camptothecin (Topotecan).
2. Higher reactivity is obtained under mild conditions and polyalkylation is minimized with electron rich substrates as effect of solvent and base shows that yield of the product decreases dramatically in the absence of a base. Herein crude product can be isolated by simple filtration.
3. Dihalomethane has double role to play when it can behave both as a solvent and a reactant for rapid reaction with dimethylamine to form Mannich adducts at atmospheric pressure.
4. Mannich products of 10-Hydroxycamptothecin have been isolated in good yield with methylene halide as a C-1 unit source, instead of formaldehyde.
5. Methylene halide and secondary amine react rapidly at room temperature and atmospheric pressure in basic conditions to form aminals (methylene-bisamines) as intermediates. In return, to our best knowledge, none of Mannich products have been obtained at atmospheric pressure, even after extended reaction times.
6. Solid -liquid transfer phase catalysis is straight forward route to desired products since use of Preformed-iminium salts (Mannich reagents) generally does not provide solution for aminoalkylation of indole, quinoline and isoquinoline alkaloids
7. Effect of solvent and base on model reaction shows that yield of the products decreases dramatically in the absence of any base or in the presence of amine like tri-butylamine. It was also observed that it is not necessary to work in anhydrous conditions using potassium carbonate as a base.
8. Methodology provides superior yields, faster reaction under milder conditions, less undesired products for preparation of complex molecules. Where conventional Mannich procedure relies on the generation of aminomethyl species through equilibria involving an amine and formaldehyde which are only suitable for aminomethylation of electron-rich aromatic species and has limited scope when extended to less reactive substrates which are inert to classical Mannich conditions.
9. Though the yields while using dibromo and diiodo methylenes were slightly higher, but keeping in view the cost and the ease with which reagents are used, dichloromethylene appears to be the best.

## Claims

1. A process for preparing 9-[(dimethylamino)-methyl]-10-hydroxycamptothecin (topotecan) of the formula 1 below 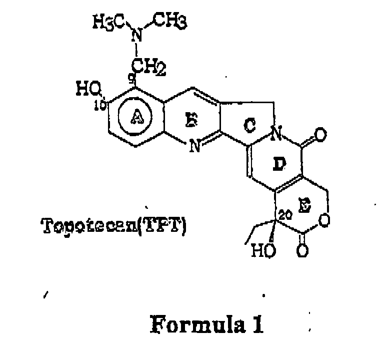 from 10-hydxoxy-20-(S)-camptothecin (HCPT) dissolved in an organic solvent, the process comprising *ortho*-regioselective aminomethylation of HCPT with dimethylamine, using a dihalomethane which behaves both as solvent and a reactant, under solid-liquid phase transfer catalysis along with a solid base catalyst in suspension form, and under stirring and at room temperature, filtering the solid product obtained and washing the obtained solid product, evaporating the solvent and purifying the residue to obtain the desired product.

2. A process as claimed in claim 1 wherein the dihalomethane is selected from the group consisting of dichloromethane, dibromomethane and diidomethane.

3. A process as claimed in claim 1 wherein the solvent medium is selected from the group consisting of methylene halides, toluene, acetonitrile, dimethylformamide and any mixture thereof.

4. A process as claimed in claim 1 wherein the solid base catalyst is selected from the group consisting of potassium carbonate, sodium carbonate, ammonium carbonate, lithium carbonate and hydrated potassium carbonate.

5. A process as claimed in claim 1 wherein the stirring is done at a pressure in the range of 69-124 kPa (10-18 psi) for a period of 4-8 hours.

6. A process as claimed in claim 1 wherein the reaction is carried out at a temperature in the range of 25 °C-45 °C and on a rotary shaker at 220-250 rpm.

7. A process as claimed in claim 1 wherein the product topotecan obtained is in the form of an acetate or a hydrochloride salt by freeze drying.

8. A process as claimed in claim 1 wherein the acetate of topotecan is converted to the pure hydrochloride salt thereof by adding dilute aqueous hydrochloric acid to the solution of acetate salt of topotecan followed by lyophilization.

9. A process as claimed in claim 1 wherein the filtered residue is washed with ethyl acetate.

10. A process as claimed in claim 1 wherein the obtained residue is purified by repeated recrystallization or by distillation.

## Patentansprüche

1. Verfahren zur Herstellung von 9-[(Dimethylamino)-methyl]-10-hydroxycamptotecin (Topotecan) mit der folgenden Formel: 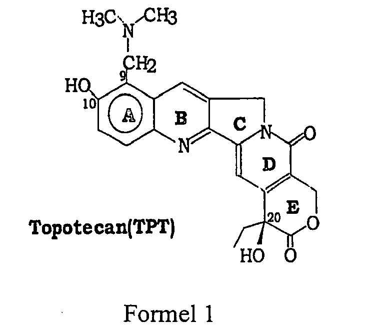 aus 10-Hydroxy-20-(S)-camptotecin (HCPT), das in einem organischen Lösungsmittel gelöst ist, wobei das Verfahren aufweist: *ortho*-regioselektive Aminomethylierung von HCPT mit Dimethylamin unter Verwendung von Dihalogenmethan, das sich wie ein Lösungsmittel und Reaktant verhält, unter Fest-Flüssig-Phasentransferkatalyse, zusammen mit einem festen Basenkatalysator in Suspensionsform und unter Rühren und bei Raumtemperatur, Filtern des erhaltenen festen Produkts und Waschen des erhaltenen festen Produkts, Verdampfen des Lösungsmittels und Reinigen des Rückstands, um das gewünschte Produkt zu erhalten.

2. Verfahren nach Anspruch 1, wobei das Dihalogenmethan aus der Gruppe ausgewählt ist, die aus Dichlormethan, Dibrommethan und Diiodmethan besteht.

3. Verfahren nach Anspruch 1, das Lösungsmittel aus der Gruppe ausgewählt ist, die aus Methylenhalogeniden, Toluol, Acetonitril, Dimethylformamid und einem Gemisch daraus besteht.

4. Verfahren nach Anspruch 1, wobei der feste Basenkatalysator aus der Gruppe ausgewählt ist, die aus Kaliumcarbonat, Natriumcarbonat, Ammoniumcarbonat, Lithiumcarbonat und hydriertem Kaliumcarbonat besteht.

5. Verfahren nach Anspruch 1, wobei das Rühren bei einem Druck im Bereich von 69-124 kPa (10-18 psi) während einer Zeitspanne von 4-8 Stunden ausgeführt wird.

6. Verfahren nach Anspruch 1, wobei die Reaktion bei einer Temperatur im Bereich von 25°C - 45°C und auf einer Umlaufschüttelmaschine bei 220-250 U/min ausgeführt wird.

7. Verfahren nach Anspruch 1, wobei das Produkt Topotecan in Form eines Acetat- oder eines Hydrochloridsalzes durch Gefriertrocknung gewonnen wird.

8. Verfahren nach Anspruch 1, wobei das Acetat von Topotecan durch Zusatz von verdünnter wäßriger Chlorwasserstoffsäure zu der Lösung des Acetatsalzes von Topotecan und anschließende Lyophilisierung in sein reines Hydrochloridsalz umgewandelt wird.

9. Verfahren nach Anspruch 1, wobei der gefilterte Rückstand mit Ethylacetat gewaschen wird.

10. Verfahren nach Anspruch 1, wobei der erhaltene Rückstand durch wiederholtes Umkristallisieren oder durch Destillieren gereinigt wird.

## Revendications

1. Procédé pour la préparation de 9-[(diméthylamino)méthyl]-10-hydroxycamptothécine (topotécan) de la formule 1 ci-dessous: 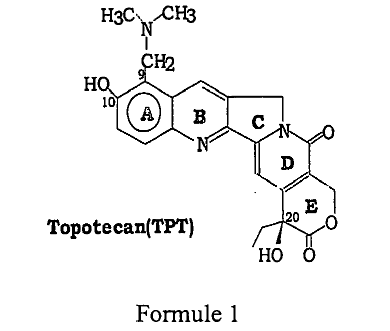 à partir de 10-hydroxy-20-(S)-camptothécine (HCPT) dissoute dans un solvant organique, le procédé comprenant une aminométhylation ortho-régiosélective de HCPT avec de la diméthylamine, en utilisant un dihalométhane qui se comporte à la fois comme un solvant et un réactif, sous une catalyse de transfert de phase solide-liquide accompagnée d'un catalyseur base solide sous forme de suspension, et sous agitation et à la température ambiante, une filtration du produit solide obtenu et un lavage du produit solide obtenu, une évaporation du solvant et une purification du résidu pour obtenir le produit désiré.

2. Procédé suivant la revendication 1, dans lequel le dihalométhane est choisi dans le groupe constitué de dichlorométhane, de dibromométhane et de diiodométhane.

3. Procédé suivant la revendication 1, dans lequel le milieu de solvant est choisi dans le groupe constitué d'halogénures de méthylène, de toluène, d'acétonitrile, de diméthylformamide et de n'importe quel mélange de ceux-ci.

4. Procédé suivant la revendication 1, dans lequel le catalyseur base solide est choisi dans le groupe constitué de carbonate de potassium, de carbonate de sodium, de carbonate d'ammonium, de carbonate de lithium et de carbonate de potassium hydraté.

5. Procédé suivant la revendication 1, dans lequel l'agitation est effectuée à une pression dans l'intervalle de 69-124 kPa (10-18 psi) pendant une période de 4-8 heures.

6. Procédé suivant la revendication 1, dans lequel la réaction est réalisée à une température dans l'intervalle de 25°C-45°C et dans un agitateur rotatif à 220-250 tpm.

7. Procédé suivant la revendication 1, dans lequel le produit de topotécan obtenu est sous la forme d'un sel d'acétate ou de chlorhydrate par une lyophilisation.

8. Procédé suivant la revendication 1, dans lequel l'acétate de topotécan est converti en sel de chlorhydrate pur de celui-ci par l'ajout d'acide chlorhydrique aqueux dilué à la solution de sel d'acétate de topotécan suivi par une lyophilisation.

9. Procédé suivant la revendication 1, dans lequel le résidu filtré est lavé avec de l'acétate d'éthyle.

10. Procédé suivant la revendication 1, dans lequel le résidu obtenu est purifié par une recristallisation répétée ou par une distillation.
